# EUROPEAN PATENT APPLICATION

(11) **EP 3 973 964 A1**
(43) Date of publication of application: **30.03.2022**
(21) Application number: 20810457.0
(22) Date of filing: 20.05.2020
(51) Int. Cl.: A61K 31/4709, A61P 35/00, A61P 35/04

(54) **QUINOLINE DERIVATIVE USED FOR COMBINATION TREATMENT OF SMALL CELL LUNG CANCER**

(30) Priority: 20.05.2019 CN 201910419546
(71) Applicant: Chia Tai Tianqing Pharmaceutical Group Co., Ltd., Lianyungang City, Jiangsu 222062 (CN)
(72) Inventor: WANG, Dong, Chongqing 400042 (CN); DAI, Nan, Chongqing 400042 (CN); LUO, Hao, Chongqing 400042 (CN); XU, Ping, Lianyungang City, Jiangsu 222062 (CN)
(74) Representative: Müller Schupfner & Partner Patent- und Rechtsanwaltspartnerschaft mbB
(86) International application number: PCT/CN2020/091312
(87) International publication number: WO 2020/233602

(57) **Abstract**

A combination pharmaceutical composition used for the treatment of small cell lung cancer, comprising: (i) compound I or a pharmaceutically acceptable salt thereof; and (ii) at least one second treatment drug, the chemical name of compound I being 1-[[[4-(4-fluoro-2-methyl-1H-indol-5-yl)oxy-6-methoxyquinolin-7-yl]oxy]methyl] cyclopropylamine.

## Description

### TECHNICAL FIELD

The present application belongs to the technical field of pharmaceuticals, and relates to use of a quinoline derivative for combating tumors. In particular, the present application relates to use of the quinoline derivative for the combination treatment of small cell lung cancer.

### BACKGROUND

Small cell lung cancer (SCLC) is the most malignant type of lung cancer, which features rapid progress, early metastasis, easy relapse and the like, and accounts for about 15-20% of new lung cancer. The occurrence of SCLC is closely related to long-term smoking. SCLC has high invasiveness, low early diagnosis rate and poor prognosis resulting from the lack of effective treatments. Untreated SCLC patients have a median survival of only 2 to 4 months from diagnosis, and a 5-year survival rate of less than 5%.

The SCLC staging system developed by the Veterans Administration Lung Study Group (VALG) has been used for a long time, and is easy to follow. According to this staging system, SCLC is briefly divided into limited disease (LD) and extensive disease (ED). The LD-SCLC is defined as a lesion confined to one side of the chest, including those with metastases in the contralateral mediastinum and bilateral supraclavicular lymph nodes, and those with ipsilateral pleural effusion, implying a complete radiation field that can be tolerated, and the ED-SCLC is defined as a lesion beyond the above range, of which the former accounts for about 30-40%. The median survival time (MST) for untreated SCLC patient is only 2-4 months; after treatment, the MST is about 15-20 months for the LD-SCLC patients and 8-13 months for the ED-SCLC patients. SCLC is more sensitive to chemotherapy and radiation therapy compared to other types of lung cancer. However, there are still many challenges in the treatment because of the high recurrence rate and drug resistance rate.

### BRIEF SUMMARY

In one aspect, the present application provides a combined pharmaceutical composition for use in treating small cell lung cancer, comprising: (i) a compound I or a pharmaceutically acceptable salt thereof; and (ii) at least one second therapeutic agent.

In another aspect, the present application also provides use of the pharmaceutical composition in preparing a medicament in treating small cell lung cancer. The present application also provides use of the pharmaceutical composition for use in treating small cell lung cancer.

In yet another aspect, the present application also provides a method for treating small cell lung cancer, comprising administering to a subject the pharmaceutical composition of the present application. The pharmaceutical composition comprises: (i) a compound I or a pharmaceutically acceptable salt thereof; and (ii) at least one second therapeutic agent.

### SUMMARY

In one aspect, the present application provides a combined pharmaceutical composition for use in treating small cell lung cancer, comprising: (i) a compound I or a pharmaceutically acceptable salt thereof; and (ii) at least one second therapeutic agent,

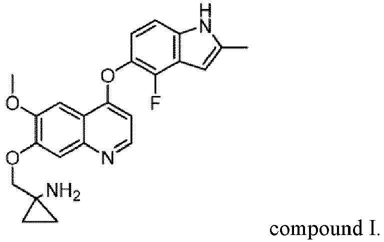

In some embodiments of the present application, the pharmaceutical composition comprises: (i) a pharmaceutical composition of the compound I or the pharmaceutically acceptable salt thereof; and (ii) a pharmaceutical composition of at least one second therapeutic agent. In some embodiments, provided is a pharmaceutical composition for use in treating small cell lung cancer, comprising: (i) the compound I or the pharmaceutically acceptable salt thereof; and (ii) at least one chemotherapeutic agent, optionally in combination with radiation therapy. In some embodiments, provided is a pharmaceutical composition for use in treating small cell lung cancer, comprising: (i) the compound I or the pharmaceutically acceptable salt thereof; and (ii) at least one small molecule targeted anti-tumor agent, optionally in combination with radiation therapy. In some embodiments, provided is a pharmaceutical composition for use in treating small cell lung cancer, comprising: (i) the compound I or the pharmaceutically acceptable salt thereof; and (ii) at least one immunotherapeutic agent, optionally in combination with radiation therapy. In some embodiments, provided is a pharmaceutical composition for use in treating small cell lung cancer, comprising: (i) the compound I or the pharmaceutically acceptable salt thereof; and (ii) at least one macromolecular antibody drug, optionally in combination with radiation therapy.

In some specific embodiments, provided is a pharmaceutical composition for use in treating small cell lung cancer, comprising: (i) the compound I or the pharmaceutically acceptable salt thereof; and (ii) a fluoropyrimidine derivative, optionally in combination with radiation therapy. In some specific embodiments, provided is a pharmaceutical composition for use in treating small cell lung cancer, comprising: (i) the compound I or the pharmaceutically acceptable salt thereof; and (ii) tegafur-gimeracil-oteracil potassium, optionally in combination with radiation therapy. In some specific embodiments, provided is a pharmaceutical composition for use in treating small cell lung cancer, comprising: (i) the compound I or the pharmaceutically acceptable salt thereof; and (ii) podophyllums and at least one platinum agent, optionally in combination with radiation therapy. In some specific embodiments, provided is a pharmaceutical composition for use in treating small cell lung cancer, comprising: (i) the compound I or the pharmaceutically acceptable salt thereof; and (ii) etoposide and at least one platinum agent, optionally in combination with radiation therapy. In some specific embodiments, provided is a pharmaceutical composition for use in treating small cell lung cancer, comprising: (i) the compound I or the pharmaceutically acceptable salt thereof; and (ii) etoposide and cisplatin, optionally in combination with radiation therapy. In some specific embodiments, provided is a pharmaceutical composition for use in treating small cell lung cancer, comprising: (i) the compound I or the pharmaceutically acceptable salt thereof; and (ii) etoposide and carboplatin, optionally in combination with radiation therapy. In some specific embodiments, provided is a pharmaceutical composition for use in treating small cell lung cancer, comprising: (i) the compound I or the pharmaceutically acceptable salt thereof; and (ii) etoposide and lobaplatin, optionally in combination with radiation therapy. In some specific embodiments, provided is a pharmaceutical composition for use in treating small cell lung cancer, comprising: (i) the compound I or the pharmaceutically acceptable salt thereof; and (ii) an anti-PD-1 antibody, optionally in combination with radiation therapy. In some specific embodiments, provided is a pharmaceutical composition for use in treating small cell lung cancer, comprising: (i) the compound I or the pharmaceutically acceptable salt thereof; and (ii) sintilimab, optionally in combination with radiation therapy. In some specific embodiments, provided is a pharmaceutical composition for use in treating small cell lung cancer, comprising: (i) the compound I or the pharmaceutically acceptable salt thereof; and (ii) camptothecin analogs, optionally in combination with radiation therapy. In some specific embodiments, provided is a pharmaceutical composition for use in treating small cell lung cancer, comprising: (i) the compound I or the pharmaceutically acceptable salt thereof; and (ii) irinotecan, optionally in combination with radiation therapy.

In another aspect, the present application provides use of a pharmaceutical composition in preparing a medicament in treating small cell lung cancer, comprising: (i) a compound I or a pharmaceutically acceptable salt thereof; and (ii) at least one second therapeutic agent, optionally in combination with radiation therapy. The present application also provides use of the pharmaceutical composition for use in treating small cell lung cancer. In yet another aspect, the present application also provides a method for treating small cell lung cancer, comprising administering to a subject the pharmaceutical composition of the present application. The pharmaceutical composition comprises: (i) a compound I or a pharmaceutically acceptable salt thereof; and (ii) at least one second therapeutic agent.

The present application provides a method for treating an entity with small cell lung cancer. In some embodiments of the present application, the entity has previously received surgical treatment, chemotherapy, and/or radiation therapy. In some specific embodiments, progressive disease recurs after the entity has achieved complete response following surgical treatment, chemotherapy, and/or radiation therapy. In some specific embodiments, the entity has failed to achieve complete response or partial response following surgical treatment, chemotherapy and/or radiation therapy.

The present application provides a method for treating small cell lung cancer, comprising administering to a patient in need thereof a compound I or a pharmaceutically acceptable salt thereof, and at least one second therapeutic agent. In some embodiments, the present application provides a method for treating small cell lung cancer that has not received chemotherapy regimens, comprising administering to a patient in need thereof the compound I or the pharmaceutically acceptable salt thereof, and at least one second therapeutic agent. In some embodiments, the present application provides a method for treating small cell lung cancer that has progressed or relapsed after receiving at least one chemotherapy, comprising administering to a patient in need thereof the compound I or the pharmaceutically acceptable salt thereof, and at least one second therapeutic agent. In some embodiments, the present application provides a method for treating small cell lung cancer that has received at least two chemotherapy regimens, comprising administering to a patient in need thereof the compound I or the pharmaceutically acceptable salt thereof, and at least one second therapeutic agent. In one embodiment, the present application provides a method for treating refractory and relapsed small cell lung cancer, comprising administering to a patient in need thereof the compound I or the pharmaceutically acceptable salt thereof, and at least one second therapeutic agent. In some embodiments, the compound I or the pharmaceutically acceptable salt thereof and at least one second therapeutic agent are used in combination for treating primary or secondary small cell lung cancer. In some embodiments, the small cell lung cancer is chemotherapy-intolerant small cell lung cancer. In some embodiments of the present application, the entity has not previously received systemic chemotherapy. In some embodiments, the entity has previously received surgical treatment, radiation therapy, induction chemotherapy, concurrent chemotherapy, and/or adjuvant chemotherapy. In some specific embodiments, the entity has not previously received systemic chemotherapy, but has received surgical treatment, radiation therapy, induction chemotherapy, concurrent chemotherapy, and/or adjuvant chemotherapy. In some specific embodiments, progressive disease recurs after the entity has achieved complete response following surgical treatment, radiation therapy, induction chemotherapy, concurrent chemotherapy and/or adjuvant chemotherapy. In some specific embodiments, the entity has failed to achieve complete response or partial response following surgical treatment, radiation therapy, induction chemotherapy, concurrent chemotherapy and/or adjuvant chemotherapy. In some specific embodiments, the cancer metastasizes after the entity has received surgical treatment, radiation therapy, induction chemotherapy, concurrent chemotherapy and/or adjuvant chemotherapy. The administration regimen can be determined according to a combination of factors such as the activity and toxicity of the drug, and tolerance of the subject. In some embodiments of the present application, for the use or method of treatment, the second therapeutic agent can be administered according to administration regimens including but not limited to, once daily (qd), every other day (qod), every 3 days (q3d), every 4 days (q4d), every 5 days (q5d), every week (q1w), every 2 weeks (q2w), every 3 weeks (q3w) or every 4 weeks (q4w), or twice daily (bid), twice weekly (biw), three times daily (tid), four times daily (qid), and the like. In some embodiments of the present application, for the use or method of treatment, the second therapeutic agent can be administered according to an intermittent administration regimen. The intermittent administration regimen includes a treatment period and an interruption period, for example, the second therapeutic agent is administered daily in the treatment period, and then interrupted in the interruption period, followed by entering the treatment period and then the interruption period. Such an intermittent administration can be repeated multiple times.

In some embodiments of the present application, for the use or method of treatment, the compound I or the pharmaceutically acceptable salt thereof can administered according to administration regimens including but not limited to, once daily at a dose of 6 mg, 8 mg, 10 mg or 12 mg, consecutive 2-week treatment and then 1-week interruption; and/or, consecutive 2-week treatment and then 2-week interruption.

In some embodiments, the second therapeutic agent and the compound I or the pharmaceutically acceptable salt thereof each have the same or different dosing cycles. In some specific embodiments, the second therapeutic agent and the compound I or the pharmaceutically acceptable salt thereof have the same dosing cycle, e.g., a 1-week, 2-week, 3-week, or 4-week dosing cycle. In some specific embodiments, the second therapeutic agent and the compound I or the pharmaceutically acceptable salt thereof have a 3-week dosing cycle.

In some embodiments, the present application provides a combined pharmaceutical composition, which is a formulation suitable for administration in a single dosing cycle (e.g., a 3-week dosing cycle), comprising 84-168 mg, preferably 112-168 mg, of the compound I or the pharmaceutically acceptable salt thereof and at least one second therapeutic agent. The compound I or the pharmaceutically acceptable salt thereof can be packaged separately in multiple aliquots (e.g., 2, 7, 14, 28 or more aliquots).

In yet another aspect, the present application provides a kit for use in treating small cell lung cancer, comprising the compound I or the pharmaceutically acceptable salt thereof and at least one second therapeutic agent each packaged separately, and optionally a package insert.

### Small cell lung cancer

In some embodiments of the present application, the small cell lung cancer is relapsed small cell lung cancer; in certain embodiments, the small cell lung cancer is refractory small cell lung cancer; in certain embodiments, the small cell lung cancer is unresectable small cell lung cancer; in certain embodiments, the small cell lung cancer is advanced small cell lung cancer. In some embodiments, the small cell lung cancer is one that has failed with the treatment of chemotherapeutic agents and/or targeted agents. In some embodiments, the small cell lung cancer is one that has received at least two chemotherapy regimens. In one embodiment, the small cell lung cancer is refractory and relapsed small cell lung cancer, wherein the "refractory and relapsed small cell lung cancer" refers to small cell lung cancer that has failed to achieve response after chemotherapy, and small cell lung cancer that has achieved response but progressed within 3 months after chemotherapy. In one embodiment, the small cell lung cancer is sensitive and relapsed small cell lung cancer. In one embodiment, the small cell lung cancer is drug-resistant and relapsed small cell lung cancer. In some embodiments, the small cell lung cancer is clinically staged, including but not limited to, locally advanced, and/or advanced (e.g., stage IIIB/IV) and/or metastatic small cell lung cancer. The metastatic small cell lung cancer includes, but is not limited to, single metastasis, disseminated metastasis and diffuse metastasis of a lesion; the metastatic lesions include, but are not limited to, lymph nodes, pleura, bone, brain, pericardium, adrenal gland, and liver; in some embodiments, the small cell lung cancer is brain metastatic small cell lung cancer. In some embodiments, the small cell lung cancer is chemotherapy-intolerant small cell lung cancer. It will be understood by those skilled in the art that a patient can also receive radiation therapy prior to, concurrently with, or subsequent to the chemotherapy.

In some embodiments, the small cell lung cancer is extensive-stage small cell lung cancer.

In some embodiments, the small cell lung cancer is limited-stage small cell lung cancer.

In some embodiments, the small cell lung cancer is metastatic small cell lung cancer, wherein the metastatic lesions include, but are not limited to, lymph nodes, pleura, bone, pericardium, adrenal gland, liver, and brain. In some embodiments, the small cell lung cancer is brain metastatic small cell lung cancer.

In some embodiments, the small cell lung cancer is refractory and relapsed small cell lung cancer that has previously received the treatment with one or more of irinotecan, platinum agent, paclitaxel and docetaxel.

In some embodiments, the small cell lung cancer is refractory and relapsed small cell lung cancer that has previously received the treatment with irinotecan and platinum agents (e.g., including but not limited to oxaliplatin, cisplatin, carboplatin, nedaplatin, dicycloplatin, lobaplatin, triplatin tetranitrate, phenanthriplatin, picoplatin, miriplatin, and satraplatin).

### Second therapeutic agent

The second therapeutic agent described herein includes, but is not limited to, a chemotherapeutic agent, a small molecule targeted anti-tumor agent, an immunotherapeutic agent, and a macromolecular antibody drug.

As used herein, the second therapeutic agents includes, but is not limited to, one or more of platinum agents, fluoropyrimidine derivatives, camptothecin analogs, taxanes, vinca alkaloids, anthracyclines, antibiotics, podophyllums, antimetabolites, and anti-tumor agents; and examples that can be listed include, but are not limited to: one or more of platinum agents (e.g., oxaliplatin, cisplatin, carboplatin, nedaplatin, dicycloplatin, lobaplatin, triplatin tetranitrate, phenanthriplatin, picoplatin, miriplatin, satraplatin), fluoropyrimidine derivatives (e.g., gemcitabine, capecitabine, ancitabine, fluorouracil, difuradin, doxifluridine, tegafur, carmofur, trifluridine, and tegafur-gimeracil-oteracil potassium), taxanes (e.g., paclitaxel, albumin-bound paclitaxel, and docetaxel), camptothecin analogs (e.g., camptothecin, hydroxycamptothecin, 9-aminocamptothecin, 7-ethylcamptothecin, irinotecan, topotecan), vinca alkaloids (vinorelbine, vinblastine, vincristine, vindesine, vinflunine), anthracyclines (epirubicin, doxorubicin, daunorubicin, pirarubicin, amrubicin, idarubicin, mitoxantrone, aclarubicin, valrubicin, zorubicin, pixantrone), pemetrexed, carmustine, melphalan, etoposide, teniposide, mitomycin, ifosfamide, cyclophosphamide, azacitidine, methotrexate, bendamustine, liposomal doxorubicin, actinomycin D (dactinomycin), bleomycin, pingyangmycin, temozolomide, dacarbazine, peplomycin, eribulin, plinabulin, sapacitabine, treosulfan, 153Sm-EDTMP, and encequidar.

In certain specific embodiments, the second therapeutic agent is one or more of platinum agents including but not limited to, cisplatin, carboplatin, nedaplatin, oxaliplatin, triplatin tetranitrate, phenanthriplatin, picoplatin, miriplatin, satraplatin, lobaplatin, and the like.

In some embodiments, the chemotherapeutic agent is selected from the group consisting of one or more of etoposide, irinotecan, cisplatin, carboplatin, lobaplatin, nedaplatin, topotecan, paclitaxel, docetaxel, temozolomide, vinorelbine, gemcitabine, cyclophosphamide, doxorubicin, vincristine, bendamustine, epirubicin, methotrexate, amrubicin, tegafur, gimeracil, oteracil, and tegafur-gimeracil-oteracil potassium.

If desired, the second therapeutic agent is used in combination with a chemotherapeutic adjuvant including but not limited to, calcium folinate (CF), leucovorin, mesna, bisphosphonate, amifostine, and hematopoietic cell colony stimulating factor (CSF). In some embodiments, the chemotherapeutic adjuvant is calcium folinate (CF), mesna, and leucovorin.

In some embodiments, the second therapeutic agent is an immunotherapeutic agent including but not limited to one or more of interferon (interferon α, interferon α-1b, interferon α-2b), interleukin, sirolimus, everolimus, ridaforolimus, and temsirolimus.

In some embodiments, the second therapeutic agent is a small molecule targeted anti-tumor agent including but not limited to protein kinase inhibitors. The protein kinase inhibitors include, but are not limited to tyrosine kinase inhibitors, serine and/or threonine kinase inhibitors, and poly ADP-ribose polymerase (PARP) inhibitors. The targets for the inhibitors include, but are not limited to, Fascin-1 protein, HDAC (histone deacetylase), proteasome, CD38, SLAMF7 (CS1/CD319/CRACC), RANKL, epidermal growth factor receptor (EGFR), anaplastic lymphoma kinase (ALK), MET gene, ROS1 gene, HER2 gene, RET gene, BRAF gene, PI3K signaling pathway, discoidin death receptor 2 (DDR2) gene, fibroblast growth factor receptor 1 (FGFR1), neurotrophic tyrosine kinase type 1 receptor (NTRK1) gene, and KRAS gene. The targets for the small molecule targeted anti-tumor agents also include cyclooxygenase-2 (COX-2), apurinic/apyrimidinic endonuclease-1 (APE1), vascular endothelial growth factor receptor (VEGFR), chemokine receptor-4 (CXCR-4), matrix metalloproteinase (MMP), insulin-like growth factor receptor (IGF-1R), Ezrin, pigment epithelium derived factor (PEDF), AS, ES, osteoprotegerin (OPG), Src, IFN, activated leukocyte cell adhesion molecule (ALCAM), HSP,JIP1, GSK-3(β (glycogen synthase kinase-3β, cell cycle regulatory protein (CyclinD1), cyclin-dependent kinase (CDK4), tissue metalloproteinase inhibitor (TIMP1), THBS3, parathyroid hormone-related protein receptor 1 (PTHR1), tumor endothelial marker 7 (TEM7), COPS3, and cathepsin K. Examples of small molecule targeted anti-tumor agents include, but are not limited to, one or more of imatinib, sunitinib, nilotinib, bosutinib, saratanib, pazopanib, trabectedin, regorafenib, cediranib, bortezomib, panobinostat, carfilzomib, ixazomib, apatinib, erlotinib, afatinib, crizotinib, ceritinib, vemurafenib, dabrafenib, cabozantinib, gefitinib, dacomitinib, osimertinib, olmutinib, alectinib, brigatinib, lorlatinib, trametinib, larotrectinib, icotinib, lapatinib, vandetanib, selumetinib, sorafenib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, rociletinib, nintedanib, lenalidomide, LOXO-292, vorolanib, bemcentinib, capmatinib, entrectinib, TAK-931, ALT-803, palbociclib, famitinib L-malate, LTT-462, BLU-667, ningetinib, tipifarnib, poziotinib, DS-1205c, capivasertib, SH-1028, DMBG, seliciclib, OSE-2101, APL-101, berzosertib, idelalisib, lerociclib, ceralasertib, PLB-1003, tomivosertib, AST-2818, SKLB-1028, D-0316, LY-3023414, allitinib, MRTX-849, AP-32788, AZD-4205, lifirafenib, vactosertib, mivebresib, napabucasin, sitravatinib, TAS-114, molibresib, CC-223, rivoceranib, CK-101, LXH-254, simotinib, GSK-3368715, TAS-0728, masitinib, tepotinib, HS-10296, AZD-4547, merestinib, olaptesed pegol, galunisertib, ASN-003, gedatolisib, defactinib, lazertinib, CKI-27, S-49076, BPI-9016M, RF-A-089, RMC-4630, AZD-3759, antroquinonol, SAF-189s, AT-101, TTI-101, naputinib, LNP-3794, HH-SCC-244, ASK-120067, CT-707, epitinib succinate, tesevatinib, SPH-1188-11, BPI-15000, copanlisib, niraparib, olaparib, veliparib, talazoparib tosylate, DV-281, siremadlin, telaglenastat, MP-0250, GLG-801, ABTL-0812, bortezomib, tucidinostat, vorinostat, resminostat, epacadostat, tazemetostat, entinostat, mocetinostat, quisinostat, LCL-161, and KML-001. In some embodiments, the small molecule targeted anti-tumor agent is one or more of sorafenib, erlotinib, afatinib, crizotinib, ceritinib, vemurafenib, dabrafenib, cabozantinib, gefitinib, dacomitinib, osimertinib, alectinib, brigatinib, lorlatinib, trametinib, larotrectinib, icotinib, lapatinib, vandetanib, selumetinib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, and nintedanib.

In some embodiments, the second therapeutic agent is a macromolecular antibody drug. The targets for the macromolecular antibody drug include, but are not limited to, any one or more of PD-1, PD-L1, cytotoxic T-lymphocyte antigen 4 (CTLA-4), platelet-derived growth factor receptor α (PDGFR-α), vascular endothelial growth factor (VEGF), human epidermal growth factor receptor-2 (HER2), epidermal growth factor receptor (EGFR), ganglioside GD2, B cell surface protein CD20, B cell surface protein CD52, B cell surface protein CD38, B cell surface protein CD319, B cell surface protein CD30 and B cell surface protein CD19/CD3.

In some embodiments, the antibody drug is an inhibitor for the interaction between the PD-1 receptor and its ligand PD-L1; in some embodiments, the antibody drug is a cytotoxic T-lymphocyte antigen 4 (CTLA-4) inhibitor. In some embodiments, the antibody drug is a platelet-derived growth factor receptor α (PDGFR-α) inhibitor.

In some embodiments, the inhibitor for the interaction between a PD-1 receptor and its ligand PD-L1 is an antibody or an antigen-binding portion thereof that binds to programmed death receptor 1 (PD-1) and/or inhibits PD-1 activity, or an antibody or an antigen-binding portion thereof that binds to programmed death ligand 1 (PD-L1) and/or inhibits PD-L1 activity, e.g., an anti-PD-1 antibody or an anti-PD-Ll antibody. In some specific embodiments, the antibody or the antigen-binding portion thereof is (a) an anti-PD-1 monoclonal antibody or an antigen-binding fragment thereof that specifically binds to human PD-1 and blocks the binding of human PD-L1 to human PD-1; or (b) an anti-PD-Ll monoclonal antibody or an antigen-binding fragment thereof that specifically binds to human PD-L1 and blocks the binding of human PD-L1 to human PD-1.

In some embodiments, the anti-PD-1 or anti-PD-Ll antibody is an anti-PD-1 or anti-PD-Ll monoclonal antibody. In some embodiments, the anti-PD-1 or anti-PD-Ll antibody is a human or murine antibody.

In some embodiments, the anti-PD-1 antibody can be selected from the group consisting of any one or more of nivolumab, pembrolizumab, durvalumab, toripalimab (JS-001), sintilimab (IBI308), camrelizumab, tislelizumab (BGB-A317), genolimzumab (GB226), lizumab (LZM009), HLX-10, BAT-1306, AK103 (HX008), AK104 (Akesobio), CS1003, SCT-I10A, F520, SG001 and GLS-010.

In some embodiments, the anti-PD-Ll antibody can be selected from the group consisting of any one or more of atezolizumab, avelumab, durvalumab, KL-A167, SHR-1316, BGB-333, JS003, STI-A1014 (ZKAB0011), KN035, MSB2311, HLX-20 and CS-1001.

In some specific embodiments, the anti-PD-1 antibody is toripalimab.

In some specific embodiments, the anti-PD-1 antibody is pembrolizumab.

In some embodiments, the inhibitor for cytotoxic T-lymphocyte antigen 4 (CTLA-4) is an anti-CTLA-4 antibody. In some specific embodiments, the anti-CTLA-4 antibody is an anti-CTLA-4 monoclonal antibody.

In some embodiments, the anti-CTLA-4 antibody can be selected from the group consisting of any one or more of ipilimumab, tremelimumab, AGEN-1884, BMS-986249, BMS-986218, AK-104 and IBI 310.

In some specific embodiments, the anti-CTLA-4 antibody is ipilimumab.

In some embodiments, the platelet-derived growth factor receptor α (PDGFR-α) inhibitor is an anti-PDGFRα antibody. In some specific embodiments, the anti-PDGFRα antibody is an anti-PDGFRα monoclonal antibody.

In some specific embodiments, the anti-PDGFRα antibody is olaratumab.

In some specific embodiments, the antibody drug can further include, but is not limited to, any one or more of bevacizumab, ramucirumab, pertuzumab, trastuzmab, cotuximab, nimotuzumab, panitumumab, necitumumab, dinutuximab, rituximab, ibritumomab, ofatumumab, obinutuzumab, alemtuzumab, daratumumab, gemtuzumab, elotuzumab, brentuximab, inotuzumab ozogamicin, and blinatumomab.

In certain specific embodiments, the second therapeutic agent is tegafur-gimeracil-oteracil potassium.

In certain specific embodiments, the second therapeutic agent is etoposide and at least one platinum agent.

In certain specific embodiments, the second therapeutic agent is one or two of etoposide and cisplatin. In a specific embodiment, the second therapeutic agent is an EP regimen (etoposide + cisplatin).

In certain specific embodiments, the second therapeutic agent is one or two of carboplatin and etoposide. In a specific embodiment, the second therapeutic agent is an EC regimen (carboplatin + etoposide).

In certain specific embodiments, the second therapeutic agent is one or two of etoposide and lobaplatin.

In certain specific embodiments, the second therapeutic agent is one, two, three or four of cyclophosphamide, vincristine, methotrexate, and etoposide. In a specific embodiment, the second therapeutic agent is a COME regimen (cyclophosphamide + vincristine + methotrexate + etoposide).

In certain specific embodiments, the second therapeutic agent is one, two or three of cyclophosphamide, doxorubicin and vincristine. In a specific embodiment, the second therapeutic agent is a CAV regimen (cyclophosphamide + doxorubicin + vincristine).

In certain specific embodiments, the second therapeutic agent is one, two or three of cyclophosphamide, doxorubicin and etoposide. In a specific embodiment, the second therapeutic agent is a CAE regimen (cyclophosphamide + doxorubicin + etoposide).

In certain specific embodiments, the second therapeutic agent is one, two or three of ifosfamide, etoposide, and cisplatin. In a specific embodiment, the second therapeutic agent is an IEP regimen (ifosfamide + etoposide + cisplatin).

In certain specific embodiments, the second therapeutic agent is one, two or three of carboplatin, paclitaxel and etoposide. In a specific embodiment, the second therapeutic agent is a CPE regimen (carboplatin + paclitaxel + etoposide).

In certain specific embodiments, the second therapeutic agent is one, two, three or four of cisplatin, vincristine, doxorubicin and etoposide. In a specific embodiment, the second therapeutic agent is a CODE regimen (cisplatin + vincristine + doxorubicin + etoposide).

In certain specific embodiments, the second therapeutic agent is topotecan.

### Compound I or pharmaceutically acceptable salt thereof

The chemical name of the compound I is 1-[[[4-(4-fluoro-2-methyl-1*H*-indol-5-yl)oxy-6-methoxyquinolin-7-yl] oxy]methyl]cyclopropylamine, which has the following structural formula:

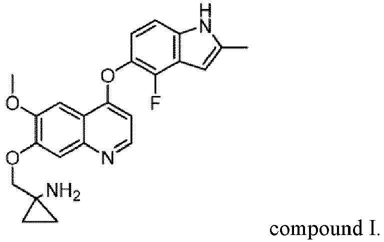

In the present application, anlotinib refers to compound I in any case.

The compound I can be administered in its free base form, or in the form of its salt, hydrate, or its prodrug that may convert *in vivo* into the free base form. For example, within the scope of the present application, the pharmaceutically acceptable salt of the compound I can be generated from various organic and inorganic acids according to methods well known in the art.

In some embodiments, the compound I or the pharmaceutically acceptable salt thereof is administered in the form of a hydrochloride salt. In some embodiments, the compound I or the pharmaceutically acceptable salt thereof is administered in the form of a monohydrochloride salt. In some embodiments, the compound I or the pharmaceutically acceptable salt thereof is administered in the form of a dihydrochloride salt. In some embodiments, the compound I or the pharmaceutically acceptable salt thereof is administered in the form of a crystalline hydrochloride salt. In a specific embodiment, the compound I or the pharmaceutically acceptable salt thereof is administered in the form of a crystalline dihydrochloride salt.

The compound I or the pharmaceutically acceptable salt thereof and the second therapeutic agent can be administered via multiple routes of administration, including but not limited to routes selected from the group consisting of oral, parenteral, intraperitoneal, intravenous, intra-arterial, transdermal, sublingual, intramuscular, rectal, transbuccal, intranasal, inhalational, vaginal, intraocular, topical, subcutaneous, intralipid, intra-articular and intrathecal routes. In a specific embodiment, the compound I or the pharmaceutically acceptable salt thereof and the second therapeutic agent is administered orally.

The amount of the compound I or the pharmaceutically acceptable salt thereof and the second therapeutic agent administered can be determined according to the severity of the disease, the response of the disease, any treatment-related toxicity, and the age and health of a subject. In some embodiments, the compound I or the pharmaceutically acceptable salt thereof is administered at a daily dose of 3 mg to 30 mg. In some embodiments, the compound I or the pharmaceutically acceptable salt thereof is administered at a daily dose of 5 mg to 20 mg. In some embodiments, the compound I or the pharmaceutically acceptable salt thereof is administered at a daily dose of 8 mg to 16 mg. In some embodiments, the compound I or the pharmaceutically acceptable salt thereof is administered at a daily dose of 10 mg to 14 mg. In some embodiments, the compound I or the pharmaceutically acceptable salt thereof is administered at a daily dose of 6 mg to 12 mg. In a specific embodiment, the compound I or the pharmaceutically acceptable salt thereof is administered at a daily dose of 6 mg. In a specific embodiment, the compound I or the pharmaceutically acceptable salt thereof is administered at a daily dose of 8 mg. In a specific embodiment, the compound I or the pharmaceutically acceptable salt thereof is administered at a daily dose of 10 mg. In a specific embodiment, the compound I or the pharmaceutically acceptable salt thereof is administered at a daily dose of 12 mg. In the present application, for example, for tablets or capsules, "comprising 12 mg of the compound I or the pharmaceutically acceptable salt thereof on a unit dose basis" means that the final tablets or capsules each comprise 12 mg of the compound I.

The compound I or the pharmaceutically acceptable salt thereof and the second therapeutic agent can be administered once or multiple times daily. In some embodiments, the compound I or the pharmaceutically acceptable salt thereof is administered once daily. In one embodiment, the compound I or the pharmaceutically acceptable salt thereof is administered once daily in the form of a solid oral formulation.

In the above methods of treatment, the administration regimen can be determined according to a combination of factors such as the activity and toxicity of the drug and tolerance of the subject. Preferably, the compound I or the pharmaceutically acceptable salt thereof is administered according to an intermittent administration regimen. The intermittent administration regimen includes a treatment period and an interruption period. The compound I or the pharmaceutically acceptable salt thereof can be administered once or multiple times daily in the treatment period. For example, the compound I or the pharmaceutically acceptable salt thereof is administered daily in the treatment period, and then interrupted in the interruption period, followed by entering the treatment period and then the interruption period. Such an intermittent administration can be repeated multiple times. The ratio of the treatment period to the interruption period in days is 2:0.5-2:5, preferably 2:0.5-2:3, more preferably 2:0.5-2:2, and even more preferably 2:0.5-2:1.

In some embodiments, the compound I or the pharmaceutically acceptable salt thereof is administered according to the administration regimen of consecutive 2-week treatment and then 2-week interruption. In some embodiments, the compound I or the pharmaceutically acceptable salt thereof is administered according to the administration regimen: once daily, consecutive 14-day treatment and then 14-day interruption; followed by once daily, consecutive 14-day treatment and then 14-day interruption. Such an intermittent administration regimen of consecutive 2-week treatment and then 2-week interruption can be repeated multiple times.

In some embodiments, the compound I or the pharmaceutically acceptable salt thereof is administered according to the administration regimen of consecutive 2-week treatment and then 1-week interruption. In some embodiments, the compound I or the pharmaceutically acceptable salt thereof is administered according to the administration regimen: once daily, consecutive 14-day treatment and then 7-day interruption; followed by once daily, consecutive 14-day treatment and then 7-day interruption. Such an intermittent administration regimen of consecutive 2-week treatment and then 1-week interruption can be repeated multiple times. In a specific embodiment, the compound I or the pharmaceutically acceptable salt thereof is administered on days 1-14 of each 21-day dosing cycle.

In some embodiments, the compound I or the pharmaceutically acceptable salt thereof is administered according to the administration regimen of consecutive 5-day treatment and then 2-day interruption. In some embodiments, the compound I or the pharmaceutically acceptable salt thereof is administered according to the administration regimen: once daily, consecutive 5-day treatment and then 2-day interruption; followed by once daily, consecutive 5-day treatment and then 2-day interruption. Such an intermittent administration regimen of consecutive 5-day treatment and then 2-day interruption can be repeated multiple times.

In certain specific embodiments, the compound I or the pharmaceutically acceptable salt thereof is administered according to the intermittent administration regimen: once daily at a dose of 12 mg, 2-week treatment and then 1-week interruption.

### Pharmaceutical combination

In certain embodiments, the compound I or the pharmaceutically acceptable salt thereof is used in combination with surgical resection and/or radiation therapy.

The components of the pharmaceutical combination described herein can be used optionally in combination with one or more pharmaceutically acceptable carriers, wherein the components can independently, or some or all of the components can together comprise a pharmaceutically acceptable carrier and/or excipient. The pharmaceutical combinations described herein can be formulated separately, or some or all of the pharmaceutical combinations can be co-formulated. Preferably, the components of the pharmaceutical composition are formulated separately or each formulated into a suitable pharmaceutical composition. In some embodiments, the pharmaceutical combination of the present application can be formulated into a pharmaceutical composition which is suitable for a single dose or multiple doses. In some specific embodiments, the pharmaceutical composition comprising the compound I or the pharmaceutically acceptable salt thereof may be selected from the group consisting of solid pharmaceutical compositions including but not limited to, tablets and capsules.

The components of the pharmaceutical combination of the present application can be administered separately, or some or all of the components are co-administered. The components of the pharmaceutical combination of the present application can be administered in a substantially asynchronous manner, or some or all of the components are administered in a substantially synchronous manner.

The components of the pharmaceutical composition of the present application can be administered independently, or some or all of the components are co-administered via various proper routes including but not limited to, oral administration or parenteral routes (intravenous, intramuscular, local or subcutaneous routes). In some embodiments, the components of the pharmaceutical combination of the present application can be administered independently, or some or all of the components are co-administered via oral administration or injection, for example, intravenous injection or intraperitoneal injection.

The components of the pharmaceutical combination of the present application can be independent suitable dosage forms, or some or all of the components are co-formulated in a suitable dosage form including but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant and dosage forms of controlled-release formulations for oral or non-oral administration.

In some embodiments of the present application, the pharmaceutical combination is a fixed combination. In some embodiments, the fixed combination is in the form of a solid pharmaceutical composition or a liquid pharmaceutical composition.

In some embodiments of the present application, the pharmaceutical combination is a non-fixed combination. In some embodiments, the second therapeutic agent and the compound I or the pharmaceutically acceptable salt thereof in the non-fixed combination are each in the form of a pharmaceutical composition.

In some embodiments of the present application, the compound I or the pharmaceutically acceptable salt thereof is administered concurrently or sequentially with one or more second therapeutic agents. In certain embodiments, the one or more second therapeutic agents have been administered to the subject prior to administration of, or combination with, the compound I or the pharmaceutically acceptable salt thereof. In certain embodiments, the one or more second therapeutic agents are administered to the subject after administration of, or combination with, the compound I or the pharmaceutically acceptable salt thereof. In certain embodiments, the compound I or the pharmaceutically acceptable salt thereof has been administered to the subject prior to administration of, or combination with, the one or more second therapeutic agents. In certain embodiments, the compound I or the pharmaceutically acceptable salt thereof is administered to the subject after administration of, or combination with, the one or more second therapeutic agents. In some embodiments, when the compound I or the pharmaceutically acceptable salt thereof is administered to a subject in combination with one or more second therapeutic agents, the compound I or the pharmaceutically acceptable salt thereof and the one or more second therapeutic agents are administered to the subject sequentially.

In some embodiments, also provided is a kit of a pharmaceutical composition for use in treating small cell lung cancer, comprising: (a) a first pharmaceutical composition comprising a chemotherapeutic agent as an active ingredient; and (b) a second pharmaceutical composition comprising the compound I or the pharmaceutically acceptable salt thereof as an active ingredient. In some embodiments, also provided is a kit of a pharmaceutical composition for use in treating small cell lung cancer, comprising: (a) a first pharmaceutical composition comprising a small molecule targeted anti-tumor agent as an active ingredient; and (b) a second pharmaceutical composition comprising the compound I or the pharmaceutically acceptable salt thereof as an active ingredient. In some embodiments, also provided is a kit of a pharmaceutical composition for use in treating small cell lung cancer, comprising: (a) a first pharmaceutical composition comprising an immunotherapeutic agent as an active ingredient; and (b) a second pharmaceutical composition comprising the compound I or the pharmaceutically acceptable salt thereof as an active ingredient. In some embodiments, also provided is a kit of a pharmaceutical composition for use in treating small cell lung cancer, comprising: (a) a first pharmaceutical composition comprising a macromolecular antibody drug as an active ingredient; and (b) a second pharmaceutical composition comprising the compound I or the pharmaceutically acceptable salt thereof as an active ingredient. In some embodiments, also provided is a kit of a pharmaceutical composition for use in treating small cell lung cancer, comprising: (a) a first pharmaceutical composition comprising tegafur-gimeracil-oteracil potassium as an active ingredient; and (b) a second pharmaceutical composition comprising the compound I or the pharmaceutically acceptable salt thereof as an active ingredient. In some embodiments, also provided is a kit of a pharmaceutical composition for use in treating small cell lung cancer, comprising: (a) a first pharmaceutical composition comprising etoposide and a platinum agent as active ingredients; and (b) a second pharmaceutical composition comprising the compound I or the pharmaceutically acceptable salt thereof as an active ingredient. In some embodiments, also provided is a kit of a pharmaceutical composition for use in treating small cell lung cancer, comprising: (a) a first pharmaceutical composition comprising etoposide and cisplatin as active ingredients; and (b) a second pharmaceutical composition comprising the compound I or the pharmaceutically acceptable salt thereof as an active ingredient. In some embodiments, also provided is a kit of a pharmaceutical composition for use in treating small cell lung cancer, comprising: (a) a first pharmaceutical composition comprising etoposide and lobaplatin as active ingredients; and (b) a second pharmaceutical composition comprising the compound I or the pharmaceutically acceptable salt thereof as an active ingredient.

### Definitions and Description

As used herein, unless otherwise stated, the following terms used in the specification and claims shall have the following meanings for the purposes of the present invention.

As used herein, the term "treat", "treating" or "treatment" generally refers to obtaining a desired pharmacological and/or physiological effect. In terms of partially or fully stabilizing or curing the disease and/or a side effect of the disease, the effect can be therapeutic. As used herein, "treat", "treating" and "treatment" encompass any treatment to a disease in a subject, including (a) inhibiting a symptom of a disease, i.e., blocking the progression of the disease; or (b) alleviating a symptom of a disease, i.e., causing the remission of the disease or the symptom.

As used herein, the term "treatment failure" or "failure with treatment" refers to intolerance of toxic and side effects, progressive disease during the treatment, or relapse after the treatment.

As used herein, the term "subject" refers to a mammal, such as a rodent, feline, canine, and primate. Preferably, the subject according to the present application is a human.

"Administer", "administering" or "administration" refers to physically introducing the composition comprising the therapeutic agent to the entity using any of a variety of methods and delivery systems known to those skilled in the art. Routes of administration include intravenous, intramuscular, subcutaneous, intraperitoneal, spinal or other parenteral routes, for example by injection or infusion. As used herein, the phrase "parenteral administration" refers to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In certain embodiments, the drug is administered via a non-parenteral route, and in certain embodiments, via oral administration. Other non-parenteral routes include local, epidermal or mucosal routes, for example, intranasal, vaginal, rectal, sublingual or local routes. Administration may also be performed, e.g., once, multiple times, and/or over one or more extended periods of time.

"Entity" includes any human or non-human animal. The term "non-human animal" includes, but is not limited to, vertebrates such as non-human primates, sheep, dogs, and rodents such as mice, rats and guinea pigs. In certain embodiments, the entity is a human. The terms "entity," "subject" and "patient" are used interchangeably herein in certain contexts.

As an example, an "anti-cancer drug" promotes cancer regression in an entity or prevents further tumor growth. In certain embodiments, the drug promotes cancer regression to the point of eliminating the cancer. "Promoting cancer regression" means that the administration of a drug, alone or in combination with an anti-tumor agent results in a reduction of tumor growth or size, necrosis of the tumor, reduction in the severity of at least one disease symptom, increase in the frequency and duration of disease symptom-free stage. Furthermore, the terms "effective" and "effectiveness" with respect to treatment include pharmacological effectiveness and physiological safety. Pharmacological effectiveness refers to the ability of a drug to promote cancer regression in a patient.

Physiological safety refers to the level of toxicity or other adverse physiological effects (adverse effects) at the cell, organ and/or organism level resulting from drug administration.

As an example for treating a tumor, an anti-cancer drug can inhibit cell growth or tumor growth by at least about 10%, at least about 20%, at least about 40%, at least about 60%, or at least about 80% relative to an untreated entity, or, in certain embodiments, relative to a subject treated with standard of care therapy. In other embodiments of the present application, tumor regression may be observed for a period of at least about 20 days, at least about 40 days, or at least about 60 days. Despite these final measurements of therapeutic effectiveness, the evaluation of drugs must also take into account "immune-related" response patterns.

An "immune-related" response pattern refers to the clinical response pattern often observed in cancer subjects treated with an immunotherapeutic agent that produces an anti-tumor effect by inducing a cancer-specific immune response or by altering the innate immune process. This response pattern is characterized by beneficial therapeutic effects following an initial increase in tumor burden or the appearance of new lesions, which would be classified as progressive disease and would be synonymous with drug failure in the evaluation of traditional chemotherapeutic agents. Thus, proper evaluation of immunotherapeutic agents may require long-term monitoring of the effect of these agents on target disease.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include a Fab fragment, a Fab' fragment, an F(ab')₂ fragment, an Fv fragment, an isolated CDR region, a single chain Fv molecule (scFv), an Fd fragment and other antibody fragments known in the art. The antibody and the fragment thereof may also include a recombinant polypeptide, a fusion protein, and a bispecific antibody. The anti-PD-Ll antibody and the fragment thereof disclosed herein can be of IgG1, IgG2, IgG3, or IgG4 isotype.

The term "isotype" refers to the class of antibodies encoded by the heavy chain constant region gene. In one embodiment, the anti-PD-1/anti-PD-L1 antibody and the fragment thereof disclosed herein are of the IgG1 or IgG4 isotype. The anti-PD-1/anti-PD-L1 antibody and the fragment thereof of the present application can be derived from any species, including but not limited to mouse, rat, rabbit, primate, llama, and human. The PD-1/PD-L1 antibody and the fragment thereof may be a chimeric antibody, a humanized antibody or an intact human antibody.

The term "humanized" means that the antigen-binding site in the antibody is derived from a non-human species and the variable region framework is derived from human immunoglobulin sequences. The humanized antibody may comprise substitutions in the framework regions such that the framework may not be an exact copy of the expressed human immunoglobulin or germline gene sequence.

The "isolated antibody" refers to an antibody that is substantially free of other antibodies having different antigenic specificities (e.g., an isolated antibody that specifically binds to PD-1/PD-L1 is substantially free of antibodies that specifically bind to antigens apart from PD-1/PD-L1). However, an isolated antibody that specifically binds to PD-1/PD-L1 may have cross-reactivity with other antigens (such as PD-1/PD-L1 molecules from different species). Furthermore, the isolated antibody may be substantially free of other cellular materials and/or chemicals.

The term "monoclonal antibody" ("mAb") refers to an antibody molecule of a single molecule composition. A monoclonal antibody composition exhibits a single binding specificity and affinity for a particular epitope or, in the case of bispecific monoclonal antibody, a dual binding specificity for two different epitopes. mAb is an example of the isolated antibody. mAbs can be produced by hybridoma techniques, recombinant techniques, transgenic techniques, or other techniques known to those skilled in the art. Examples of isolated monoclonal antibodies include, but are not limited to, nivolumab (Opdivo^{®}), pembrolizumab (Keytruda^{®}), durvalumab, avelumab, toripalimab (JS-001, Junshi Biosciences), sintilimab (IBI308, Innovent Biologies), camrelizumab (SHR-1210, Hengrui Medicine, refer to CN105026428B or WO2015085847A1), tislelizumab (BGB-A317, BeiGene), genolimzumab (GB226, Genor Biopharma), lizumab (LZM009, Livzon), HLX-10 (Henlius), BAT-1306 (Bio-Thera), HX008 (AK103, Akeso Bioscience/Hanzhong Pharmaceuticals), AK104 (Akeso Bioscience), CS1003 (CStone Pharmaceuticals), SCT-l10A (SinoCellTech), F520 (Shandong New Time Pharmaceutical/Lunan Pharmaceutical Group), SG001 (Sumgen Bio), GLS-010 (Goloria Pharceuticals), atezolizumab (Tecentriq^{®}, Roche), avelumab (Bavencio^{®}, Merck/Pfizer), durvalumab (Imfinzi^{®}, AstraZeneca), KL-A167 (Kelun Pharmaceutical), SHR-1316 (Hengrui Medicine), BGB-333 (BeiGene), JS003 (Junshi Biosciences), STI-A1014 (ZKAB0011, Zhaoke Pharmaceutical), KN035 (Alphamab Oncology/3D Medicines ), MSB2311 (Mabspace Biosciences), HLX-20 (Henlius), CS-1001 (CStone Pharmaceuticals), etc.

An "antigen-binding portion" (also referred to as an "antigen-binding fragment") of an antibody refers to one or more fragments of the antibody that retain the ability to specifically bind to an antigen to which an intact antibody binds.

"Programmed death receptor-1 (PD-1)" refers to an immunosuppressive receptor belonging to the CD28 family. PD-1 is expressed primarily on previously activated T cells *in vivo* and binds to two ligands PD-L1 and PD-L2. As used herein, the term "PD-1" includes human PD-1 (hPD-1), variants, homologs and species homologs of hPD-1, and analogs having at least one common epitope with hPD-1.

"Programmed death ligand-1 (PD-L1)" is one of two cell surface glycoprotein ligands for PD-1 (the other is PD-L2), which down-regulates T cell activation and cytokine secretion upon binding to PD-1.

A "recurrent" cancer is one that regenerates at the initial site or a distant site after being responsive to initial treatment (e.g., surgery). A "locally recurrent" cancer is one that occurs, after treatment, at the same location as the previously treated cancer.

An "unresectable" cancer is one that cannot be removed by surgery.

A "metastatic" cancer refers to one that spreads from one part of the body (e.g., the lung) to another part of the body.

The use of alternatives (e.g., "or") shall be understood to refer to any one, two, or any combination of the alternatives. As used herein, the indefinite article "a" or "an" shall be understood to mean "one or more" of any listed or enumerated components.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed by basic radicals and free acids and salts formed by acidic radicals and free bases, for example, hydrochloride, hydrobromide, nitrate, sulfate, phosphate, formate, acetate, trifluoroacetate, fumarate, oxalate, maleate, citrate, succinate, mesylate, benzenesulfonate, p-methylbenzenesulfonate, sodium salt, potassium salt, ammonium salt or amino acid salt, preferably, hydrochloride, hydrobromide, sulfate, formate, acetate, trifluoroacetate, fumarate, maleate, mesylate, p-methylbenzenesulfonate, sodium salt, potassium salt, ammonium salt, and amino acid salt, etc. In the present application, when forming a pharmaceutically acceptable salt, the free acid and the basic radical are in a molar ratio of about 1:0.5 to 1:5, preferably 1:0.5, 1:1, 1:2, 1:3, 1:4, 1:5, 1:6, 1:7 or 1:8. In the present application, when forming a pharmaceutically acceptable salt, the free base and the acidic radical are in a molar weight ratio of about 1:0.5 to 1:5, preferably 1:0.5, 1:1, 1:2, 1:3, 1:4, 1:5, 1:6, 1:7 or 1:8.

The term "fixed combination" refers to administration of the active components (for example, the chemotherapeutic agent or the compound I) to a subject simultaneously at a fixed total dose or in a fixed dose proportion, or in the form of a single substance, pharmaceutical composition or formulation.

The term "non-fixed combination" refers to simultaneous, parallel, or sequential and temporally unlimited administration of two or more aforementioned active components as independent substances (for example, a pharmaceutical composition and a formulation) to a subject, wherein the active ingredients administered to the subject reach therapeutically effective amounts. An example, which can be listed, of the non-fixed combination is a cocktail therapy, for example, 3 or more active components are administered. In a non-fixed combination, each active component can be packaged, sold or administered as a fully independent pharmaceutical composition. The term "non-fixed combination" also includes combined use of "fixed combinations", or a "fixed combination" and an independent substance of any one or more active components.

As used herein, "combined use" or "use in combination" means that two or more active substances may be administered to a subject as a mixture, simultaneously as a single formulation, or sequentially in any order as a single formulation.

The term "pharmaceutical composition" refers to a mixture consisting of one or more of the active ingredients (e.g, the second therapeutic agent or the compound I) or pharmaceutical combinations thereof of the present application and a pharmaceutically acceptable excipient. The pharmaceutical composition is intended to facilitate the administration of the compound or the pharmaceutical combination thereof to a subject.

The "clinical benefits" in the present application include, but are not limited to: prolonged progression-free survival (PFS), prolonged overall survival (OS), improved objective response rate (ORR), improved disease control rate (DCR), reduced number and/or degree of adverse effects for clinical patients.

In the present application, "about" refers to the fluctuation within ±5%, preferably within ±2%, and more preferably within ±1% of the specified numerical range given.

As used herein, unless otherwise stated, the terms "comprise", "comprises" and "comprising" or equivalents thereof are open-ended statements and mean that elements, components and steps that are not specified may be included in addition to those listed.

All patents, patent applications and other identified publications are expressly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates of these documents or the content of these documents. Moreover, in any country or region, any reference to these publications herein is not to be construed as an admission that the publications form part of the commonly recognized knowledge in the art.

### DETAILED DESCRIPTION

The present application is further described below with reference to specific examples, which are, however, only for illustration and do not limit the scope of the present application. Likewise, the present application is not limited to any specific preferred embodiment described herein. It should be understood by those skilled in the art that equivalent substitutions and corresponding modifications of the technical features of the present application still fall within the protective scope of the present application. Unless otherwise stated, the reagents used in the following examples are commercially available products, and the solutions can be prepared by conventional techniques in the art.

### Example 1

Patients who were pathologically diagnosed with small cell lung cancer (including ED-SCLC and LD-SCLC) with measurable lesions were divided into the following groups: two combination administration groups adopting a combination of either EP or EC regimen (etoposide + cisplatin/carboplatin) + anlotinib, and a control group adopting either EP or EC alone (etoposide + cisplatin/carboplatin). The administration regimens are detailed below.

In the combination administration group, patients received etoposide: 100 mg/m², i.v., for 120 min, administered at d1-3 of each 21-day dosing cycle; cisplatin: 75 mg/m², or carboplatin: AUC 5, i.v., for 120 min, administered at d1 of each 21-day dosing cycle; anlotinib, 12 mg/day, administered orally on an empty stomach in the morning at d1-14 of each 21-day dosing cycle. After 4-6 dosing cycles of combination treatment, anlotinib was used for maintenance treatment: 12 mg/day, administered orally on an empty stomach in the morning at d1-14 of each 21-day dosing cycle until progressive disease.

In the control group, patients received etoposide: 100 mg/m², i.v., for 120 min, administered at d1-3 of each 21-day dosing cycle; cisplatin: 75 mg/m² or carboplatin: AUC 5, i.v., for 120 min, administered at d1 of each 21-day dosing cycle.

The observation indexes are as follows: primary endpoint: progression-free survival (PFS); secondary endpoint: objective response rate (ORR), overall survival (OS), disease control rate (DCR), safety, and tolerability.

The clinical trial was implemented, and 31 patients were enrolled between February 2018 and October 2019. As of now, the efficacy evaluation results of the combination administration groups were as follows: the median PFS was 8.9 months, 3 patients (3/31) with complete response (CR), 20 patients (20/31) with partial response (PR), 9 patients (9/31) with stable disease (SD), 0 patients (0/31) with no progressive disease (PD), 74.2% (21/31) in objective response rate (ORR), and 100% (31/31) in disease control rate (DCR). It was found that the combination of anlotinib and EP or EC regimen had good clinical efficacy and safety.

### Example 2

Patients who have been histopathologically and cytologically diagnosed with small cell lung cancer, have previously received 1 systemic chemotherapy adopting platinum-based chemotherapy regimens, and relapsed or failed after treatment, excluding patients with non-small cell lung cancer (including small cell and non-small cell mixed types), were divided into a combination administration group and a single-drug chemotherapy control group. The administration regimens are detailed below.

Combination administration group: anlotinib hydrochloride capsule 12 mg qd po d1-14 and irinotecan 60-80 mg/m² i.v. d1 and d8; q3w, no more than 6 chemotherapy dosing cycles.

Single-drug chemotherapy control group: irinotecan 60-80 mg/m² i.v. d1 and d8; q3w, no more than 6 chemotherapy dosing cycles.

Patients who achieved complete response (CR), partial response (PR), and stable disease (SD) continued to be administered until progressive disease (PD), intolerable toxicity, or patients who required withdrawal or achieved progressive disease (PD) were discontinued. The efficacy evaluation observation indexes include progression-free survival (PFS), overall survival (OS), objective response rate (ORR), disease control rate (DCR), duration of response (DOR), quality of life score and drug safety.

This study showed that, for patients with small cell lung cancer, the administration regimen of anlotinib in combination with irinotecan had clinical benefits.

### Example 3

Patients with extensive-stage small cell lung cancer after the pathological diagnosis of small cell lung cancer and failure with second-line chemotherapy (having received two previous chemotherapy regimens) were administered with anlotinib in combination with etoposide. The administration regimens are detailed below.

Etoposide soft capsules: once daily, oral administration before breakfast, 100 mg (by weight of etoposide comprised therein) each time, administered at d1-10 of each 21-day dosing cycle.

Anlotinib hydrochloride capsules: once daily, oral administration before breakfast, 12 mg each time, administered at d1-14 of each 21-day dosing cycle.

The interval between the two drugs was more than half an hour. If there was a missed dose, the interval needed to be confirmed that it was not shorter than 12 hours before the next dose, otherwise no more doses would be taken. During the administration, dosage adjustments could be made according to the degree of drug-related toxicity and possible therapeutic benefit of the patient. The dose could be adjusted to the following 2 levels for anlotinib: 10 mg and 8 mg. If a reduction of more than 2 dose levels was required, the treatment was discontinued. The dose of etoposide could be reduced to 50 mg.

The efficacy evaluation observation indexes include progression-free survival (PFS), overall survival (OS), objective response rate (ORR = CR + PR), disease control rate (DCR = CR + PR + SD) and drug safety.

This study showed that, for patients with small cell lung cancer, the administration regimen of anlotinib in combination with etoposide had clinical benefits.

### Example 4

Patients who have been histologically or cytologically diagnosed with small cell lung cancer (not limited to LD-SCLC or ED-SCLC), have previously received at least one first-line standard platinum-based therapy for SCLC and have progressed on objective imaging, include patients who have:
[1] sensitive relapse: progression ≥ 90 days from the last chemotherapy,
[2] drug-resistant relapse: progression during chemotherapy or < 90 days from the last chemotherapy;

The combination administration of anlotinib and S-1 (tegafur-gimeracil-oteracil potassium), as well as S-1 maintenance treatment was performed in 3-week dosing cycle for 6 dosing cycles, with the two drugs administered sequentially on day 1 of each dosing cycle, with an administration time window of ± 3 days. Within 3 days prior to each administration, the subjects had to complete various examinations including vital signs, physical examination, laboratory examination, physical status score and the like, in addition to the imaging examination. The subjects were also required to complete a tumor imaging evaluation (consistent evaluation protocol throughout the treatment, CT or MRI) within 48 weeks after the first administration at a frequency of once every 6 weeks (±7 days) and 48 weeks after the first administration at a frequency of once every 9 weeks (±7 days). The administration regimens are detailed below.

Combination treatment (6 dosing cycles):
Anlotinib hydrochloride capsules 12 mg qd and S-1 60 mg/m² bid, consecutive 2-week treatment and 1-week interruption.

Maintenance treatment:
After completion of induction treatment, subjects continued to receive S-1 maintenance treatment (20 mg/m² bid, consecutive 2-week treatment and 1-week interruption, in a 21-day dosing cycle) until progressive disease, intolerable toxic and side effects, withdrawal of informed consent or investigator's decision to withdrawal the patient from the study, noncompliance with the study treatment or procedure, or other reasons specified in the protocol.

The following evaluation indexes of S-1 in combination with anlotinib hydrochloride for treating relapsed small cell lung cancer were evaluated: progression-free survival (PFS) and objective response rate (ORR), overall survival (OS); disease control rate (DCR), PFS rate at 6 months and 1 year, OS rate at 1 year and 2 years, safety, and the like.

### Example 5

Patients who have been histologically or cytologically confirmed pathologically diagnosed with small cell lung cancer, have previously received at least one systemic chemotherapy regimen and have relapsed were administered with anlotinib in combination with sintilimab. The administration regimens are detailed below. Anlotinib hydrochloride capsules: once daily, oral administration before breakfast, 12 mg each time, administered at d1-14 of each 21-day dosing cycle, consecutive 2-week treatment and 1-week interruption.

Sintilimab: 200 mg, i.v. infusion, administered d1 of each 21-day dosing cycle.

All patients continued for a treatment period of up to 12 months, or until progressive disease, development of an intolerable toxic response, withdrawal of informed consent, death, or other event requiring discontinuation of treatment specified in the protocol, whichever occurred first.

The efficacy evaluation observation indexes include progression-free survival (PFS), overall survival (OS), objective response rate (ORR = CR + PR), disease control rate (DCR = CR + PR + SD) and drug safety. This study showed that, for patients with small cell lung cancer, the administration regimen of anlotinib in combination with sintilimab had clinical benefits, with prolonged progression-free survival, with patients having prolonged progression-free survival of up to 6 months to date.

In the above examples of the present application, the amount of anlotinib hydrochloride capsule was by weight of the free base of anlotinib comprised therein.

According to the content disclosed in the present application, the compositions and methods of the present application have been described in terms of preferred embodiments. However, it will be apparent to those skilled in the art that variations may be applied to the compositions and/or methods and the steps or the sequence of steps of the methods described herein without departing from the concept, spirit and scope of the present application.

The disclosed contents of all documents cited herein are hereby incorporated by reference to the extent that they provide exemplary, procedural and other details supplementary to those described herein.

## Claims

1. A combined pharmaceutical composition for use in treating small cell lung cancer, comprising: (i) a compound I or a pharmaceutically acceptable salt thereof; and (ii) at least one second therapeutic agent,

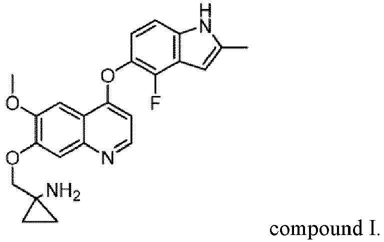

2. The pharmaceutical composition according to claim 1, wherein the small cell lung cancer is relapsed, and/or refractory, and/or unresectable, and/or advanced, and/or metastatic small cell lung cancer, preferably small cell lung cancer that has failed with the treatment of chemotherapeutic agents and/or targeted agents, small cell lung cancer that has received at least two chemotherapy regimens, refractory and relapsed small cell lung cancer, locally advanced and/or advanced metastatic small cell lung cancer.

3. The pharmaceutical composition according to claim 1 or 2, wherein the second therapeutic agent is one or more of a chemotherapeutic agent, a small molecule targeted anti-tumor agent, an immunotherapeutic agent, and a macromolecular antibody drug.

4. The pharmaceutical composition according to claim 3, wherein the chemotherapeutic agent is one or more of oxaliplatin, cisplatin, carboplatin, nedaplatin, dicycloplatin, lobaplatin, triplatin tetranitrate, phenanthriplatin, picoplatin, miriplatin, satraplatin, gemcitabine, capecitabine, ancitabine, fluorouracil, difuradin, doxifluridine, tegafur, carmofur, trifluridine, paclitaxel, albumin bound paclitaxel and docetaxel, camptothecin, hydroxycamptothecin, 9-aminocamptothecin, 7-ethylcamptothecin, irinotecan, topotecan, vinorelbine, vinblastine, vincristine, vindesine, vinflunine, epirubicin, doxorubicin, daunorubicin, pirarubicin, amrubicin, idarubicin, mitoxantrone, aclarubicin, valrubicin, zorubicin, pixantrone, pemetrexed, carmustine, melphalan, etoposide, teniposide, mitomycin, ifosfamide, cyclophosphamide, azacitidine, methotrexate, bendamustine, liposomal doxorubicin, actinomycin D, bleomycin, pingyangmycin, temozolomide, dacarbazine, peplomycin, eribulin, plinabulin, sapacitabine, treosulfan, 153Sm-EDTMP, tegafur-gimeracil-oteracil potassium and encequidar.

5. The pharmaceutical composition according to claim 3, wherein the immunotherapeutic agent is one or more of interferon, interleukin, sirolimus, everolimus, ridaforolimus, and temsirolimus.

6. The pharmaceutical composition according to claim 3, wherein the small molecule targeted anti-tumor agent is one or more of imatinib, sunitinib, nilotinib, bosutinib, saratanib, pazopanib, trabectedin, regorafenib, cediranib, bortezomib, panobinostat, carfilzomib, ixazomib, apatinib, erlotinib, afatinib, crizotinib, ceritinib, vemurafenib, dabrafenib, cabozantinib, gefitinib, dacomitinib, osimertinib, olmutinib, alectinib, brigatinib, lorlatinib, trametinib, larotrectinib, icotinib, lapatinib, vandetanib, selumetinib, sorafenib, olmutinib, savolitinib, fruquintinib, entrectinib, dasatinib, ensartinib, lenvatinib, itacitinib, pyrotinib, binimetinib, erdafitinib, axitinib, neratinib, cobimetinib, acalabrutinib, famitinib, masitinib, ibrutinib, rociletinib, nintedanib, lenalidomide, LOXO-292, vorolanib, bemcentinib, capmatinib, entrectinib, TAK-931, ALT-803, palbociclib, famitinib L-malate, LTT-462, BLU-667, ningetinib, tipifarnib, poziotinib, DS-1205c, capivasertib, SH-1028, DMBG, seliciclib, OSE-2101, APL-101, berzosertib, idelalisib, lerociclib, ceralasertib, PLB-1003, tomivosertib, AST-2818, SKLB-1028, D-0316, LY-3023414, allitinib, MRTX-849, AP-32788, AZD-4205, lifirafenib, vactosertib, mivebresib, napabucasin, sitravatinib, TAS-114, molibresib, CC-223, rivoceranib, CK-101, LXH-254, simotinib, GSK-3368715, TAS-0728, masitinib, tepotinib, HS-10296, AZD-4547, merestinib, olaptesed pegol, galunisertib, ASN-003, gedatolisib, defactinib, lazertinib, CKI-27, S-49076, BPI-9016M, RF-A-089, RMC-4630, AZD-3759, antroquinonol, SAF-189s, AT-101, TTI-101, naputinib, LNP-3794, HH-SCC-244, ASK-120067, CT-707, epitinib succinate, tesevatinib, SPH-1188-11, BPI-15000, copanlisib, niraparib, olaparib, veliparib, talazoparib tosylate, DV-281, siremadlin, telaglenastat, MP-0250, GLG-801, ABTL-0812, bortezomib, tucidinostat, vorinostat, resminostat, epacadostat, tazemetostat, entinostat, mocetinostat, quisinostat, LCL-161, and KML-001.

7. The pharmaceutical composition according to claim 3, wherein the macromolecular antibody drug is any one or more of an anti-PD-1 antibody, an anti-PD-Ll antibody, an anti-CTLA-4 antibody, an anti-PDGFRα antibody, bevacizumab, ramucirumab, pertuzumab, trastuzmab, cotuximab, nimotuzumab, panitumumab, necitumumab, dinutuximab, rituximab, ibritumomab, ofatumumab, obinutuzumab, alemtuzumab, daratumumab, gemtuzumab, elotuzumab, brentuximab, inotuzumab ozogamicin, and blinatumomab.

8. The pharmaceutical composition according to claim 7, wherein the anti-PD-1 antibody is selected from the group consisting of any one or more of nivolumab, pembrolizumab, durvalumab, toripalimab, sintilimab, camrelizumab, tislelizumab, genolimzumab, lizumab, HLX-10, BAT-1306, AK103, AK104, CS1003, SCT-I10A, F520, SG001, and GLS-010; the anti-PD-Ll antibody is selected from the group consisting of any one or more of atezolizumab, avelumab, durvallumab, KL-A167, SHR-1316, BGB-333, JS003, STI-A1014, KN035, MSB2311, HLX-20 and CS-1001; the anti-CTLA-4 antibody is selected from the group consisting of any one or more of ipilimumab, tremelimumab, AGEN-1884, BMS-986249, BMS-986218, AK-104 and IBI 310.

9. The pharmaceutical composition according to claim 3, wherein the second therapeutic agent is one or more of fluoropyrimidine derivatives, podophyllums, platinum agents, camptothecin analogs, and anti-PD-1 antibodies.

10. The pharmaceutical composition according to any one of claims 1 to 8, wherein the second therapeutic agent is any one or more of the following (1) to (14):
(1) tegafur-gimeracil-oteracil potassium;
(2) etoposide and at least one platinum agent;
(3) one, two, three or four of cyclophosphamide, vincristine, methotrexate and etoposide;
(4) one, two or three of cyclophosphamide, doxorubicin and vincristine;
(5) one, two or three of cyclophosphamide, doxorubicin and etoposide;
(6) one, two or three of ifosfamide, etoposide and cisplatin;
(7) one, two or three of carboplatin, paclitaxel and etoposide;
(8) one, two, three or four of cisplatin, vincristine, doxorubicin and etoposide;
(9) topotecan;
(10) etoposide and lobaplatin;
(11) etoposide and cisplatin;
(12) etoposide and carboplatin;
(13) sintilimab; and
(14) irinotecan.

11. The pharmaceutical composition according to any one of claims 1 to 10, wherein the compound I or the pharmaceutically acceptable salt thereof is administered at a daily dose of 3 mg to 30 mg, preferably 5 mg to 20 mg, more preferably 8 mg to 16 mg, further preferably 8 mg to 14 mg, and most preferably 8 mg, 10 mg, or 12 mg.

12. The pharmaceutical composition according to any one of claims 1 to 11, wherein the compound I or the pharmaceutically acceptable salt thereof is administered on days 1-14 of each 21-day dosing cycle.

13. Use of the pharmaceutical composition according to any one of claims 1 to 12 in preparing a medicament for treating small cell lung cancer.

14. A kit for use in treating small cell lung cancer, comprising: (a) a first pharmaceutical composition comprising a chemotherapeutic agent and/or a small molecule targeted anti-tumor agent and/or an immunotherapeutic agent and/or a macromolecular antibody drug as an active ingredient; and (b) a second pharmaceutical composition comprising the compound I or the pharmaceutically acceptable salt thereof according to any one of claims 1 to 12, as an active ingredient.

15. A method for treating small cell lung cancer, comprising administering to a subject in need thereof the pharmaceutical composition according to any one of claims 1 to 12.
